# FASCICULE DE BREVET EUROPEEN

(11) **EP 1 682 065 B1**
(45) Date de publication et mention de la délivrance du brevet: **01.10.2008**
(21) Numéro de dépôt: 04805449.8
(22) Date de dépôt: 12.11.2004
(51) Int. Cl.: A61H 1/00, A61G 15/00

(54) **Fauteuil de consultation**
Untersuchungsstuhl
Examination chair

(30) Priorité: 14.11.2003 FR 0313367; 12.07.2004 FR 0407743
(43) Date de publication de la demande: 26.07.2006
(73) Titulaire: Richard-Vitton, Thomas, 13122 Ventabren (FR)
(72) Inventeur: Richard-Vitton, Thomas, 13122 Ventabren (FR)
(74) Mandataire: Pouillot, Laurent Pierre Paul
(86) Numéro de dépôt international: PCT/FR2004/002909
(87) Numéro de publication internationale: WO 2005/048907

(56) Documents cités:
- FR-A- 1 113 809
- US-A- 3 716 046
- US-A- 4 402 500
- FURMAN J M ET AL: "TREATMENT OF BENIGN POSITIONAL VERTIGO USING HEELS-OVER-HEAD ROTATION" ANNALS OF OTOLOGY, RHINOLOGY AND LARYNGOLOGY, ANNALS PUBL., ST. LOUIS, MO, US, vol. 107, 1998, pages 1046-1053, XP008016037 ISSN: 0003-4894

## Description

La présente invention concerne un fauteuil de consultation permettant notamment à un praticien de traiter les vertiges positionnels. Ainsi, ce fauteuil autorise la mobilisation d'une personne assise, selon un mode multidirectionnel et sur une grande amplitude, utile dans la prise en charge de ces vertiges.

L'oreille interne d'un être humain permet au cerveau de ce dernier d'enregistrer les mouvements qu'il subit par l'intermédiaire de trois canaux semi-circulaires orientés selon trois plans sensiblement perpendiculaires. En outre, l'organe vestibulaire récepteur de l'oreille interne comprend une zone renseignant le cerveau sur les phénomènes d'accélérations/décélération, constituée en partie d'une masse gélatineuse lestée par de petits cristaux dénommés « otolithes » par l'homme du métier. Les vertiges positionnels peuvent alors être provoqués par les phénomènes suivants qui induisent une perception erronée de mouvement :
- la cupulolithiase provoquée par des otolithes qui se détachent puis migrent au niveau de la cupule qui est une partie très sensible d'un canal semi-circulaire, ou
- la canalolithiase provoquée par des otolithes qui se détachent et qui circulent dans un canal semi-circulaire.

Au cours de la phase de diagnostic, le praticien déplace lentement le tronc et la tête du patient, sur 180°, dans trois plans : d'avant en arrière, latéralement depuis un côté vers l'autre et ensuite en rotation dans l'axe du corps.

Si un vertige positionnel est diagnostiqué, le praticien déplace le tronc et la tête du patient, selon le plan concerné, rapidement avec une décélération brusque. Ainsi, les otolithes à l'origine du vertige sont expulsés de la zone sensible et le vertige est généralement guéri en une à trois séances.

On connaît un fauteuil de consultation qui présente une mobilité autour d'un axe vertical, permettant une rotation du patient assis. Il est possible de réaliser une bascule du dossier afin d'allonger le patient et il existe également une possibilité d'élever le fauteuil par rapport à son socle, le plus souvent par un système à crémaillère à commande électrique ou encore, un système hydraulique à commande à pied. Ces mobilités du fauteuil sont largement suffisantes pour la plupart des consultations. En revanche, les manipulations diagnostique et thérapeutique relatives aux vertiges positionnels ne sont pas toutes réalisables puisque la mobilité n'est possible que selon un unique plan et elles sont de plus pénibles pour le patient et le praticien.

On connaît, par le document FR 1 113 809, un appareil pour la rééducation et le contrôle des réflexes comportant un support oscillant, de préférence à plusieurs degrés de liberté de mouvement, sur lequel le patient est placé de manière à pouvoir amortir ou amplifier les oscillations du support par déplacement de son centre de gravité.

Ce document est éloigné du domaine technique des traitements des vertiges. En effet, le but du dispositif décrit est de stimuler les réflexes d'une personne handicapée et non pas de déplacer des otolithes de l'oreille interne qui provoquent des vertiges positionnels. En outre, il ne permet nullement une mise en mouvement rapide suivie d'une décélération brusque.

Les manipulations diagnostique et thérapeutique liées au traitement des vertiges sont donc impossibles à réaliser de manière satisfaisante avec un dispositif de type connu.

Elles sont donc généralement pratiquées sur un divan d'examen et nécessitent beaucoup de force physique et de savoir faire de la part du praticien, ainsi qu'une excellente coopération de la part des patients. Or, celle-ci ne peut être obtenue chez les patients arthrosiques, obèses, handicapés ou très angoissés, qui sont par conséquent particulièrement difficiles à prendre en charge ce qui génère des échecs thérapeutiques.

De plus, la nécessité de posséder, au sein d'un cabinet médical, d'un divan d'examen en plus d'un fauteuil de consultation augmente considérablement l'encombrement ainsi que les coûts inhérents à l'installation du cabinet.

La présente invention a alors pour objet un fauteuil de consultation médicale qui permet de mobiliser un patient, selon trois plans perpendiculaires, rapidement et sur une grande amplitude, jusqu'à 180 degrés et plus, tout en autorisant une décélération brusque à l'issue du mouvement.

Selon l'invention, un fauteuil de consultation médicale, pour asseoir et mobiliser un patient selon trois plans sensiblement perpendiculaires et sur une grande amplitude, comporte un arc primaire relié à une colonne immobile via un arbre horizontal qui constitue un premier axe de rotation, un deuxième axe de rotation, sensiblement perpendiculaire au premier axe de rotation, passant par une première et une deuxième extrémités dudit arc primaire. De plus, ce fauteuil comporte un arc secondaire, muni d'une assise, qui est agencé à l'intérieur de l'arc primaire en étant respectivement fixé par une troisième et une quatrième extrémité aux première et deuxième extrémités via un arbre supérieur et un arbre inférieur, disposés selon le deuxième axe de rotation, les arcs primaire et secondaire étant alors aptes à respectivement effectuer un mouvement rotatif autour des premier et deuxième axes de rotation. L'invention est notamment remarquable en ce qu'elle comporte un moyen de freinage pour arrêter brusquement le mouvement rotatif.

Avantageusement, l'arc primaire est relié à l'arbre horizontal par le milieu de sa convexité.

Ainsi, le praticien est à même de mouvoir un patient disposé sur l'assise en rotation autour de l'axe de son corps, latéralement depuis un côté vers l'autre et d'avant en arrière. En outre, le moyen de freinage permet de générer la décélération brusque qu i permettra d'évacuer d'éventuels otolithes gênants.

D'autre part, le patient étant mis en mouvement en « bloc » par le fauteuil, les problèmes liés au manque de mobilité ou de coopération du patient sont supprimés. Le confort de ce dernier est ainsi augmenté et les manipulations réalisées sont optimalisées et donc plus efficaces.

Pour minimiser les efforts à fournir par le praticien pou r mettre en mouvement l'arc primaire et/ou l'arc secondaire, il est impératif que le centrage soit correct, c'est-à-dire que le centre de gravité du patient soit le plus proche possible de l'intersection des premier et deuxième axes de rotation. Dans cette optique, la position verticale de l'arbre horizontal sur la colonne immobile est réglable, à l'aide d'un système de glissière à crémaillère par exemple. Identiquement, l'arc secondaire peut être déplacé selon le premier axe de rotation, via un moyen usuel agissant sur l'arbre inférieur et/ou supérieur, pour participer au réglage du centrage.

De même, la position de l'assise le long du deuxième axe de rotation est réglable à l'aide d'un premier vérin, hydraulique ou électrique par exemple.

Avantageusement, le fauteuil de consultation selon l'inventio n comporte un premier et un deuxième moteur pour respectivement mettre en rotation l'arc primaire autour du premier axe de rotation et l'arc secondaire autour du deuxième axe de rotation. De ce fait, le praticien n'a plus d'efforts à fournir pour mobiliser le patient ce qui facilite évidemment son travail.

Selon un premier mode de réalisation entièrement motorisé, le moyen de freinage inclut les premier et deuxième moteurs. En effet, en étant fortement démultipliés, ces premier et deuxième moteurs peuvent être immobilisés brusquement et donc remplir la fonction requise du moyen de freinage.

Selon un deuxième mode de réalisation, le moyen de freinage comporte un premier frein hydraulique ou électrique, par manque de courant par exemple, pour immobiliser brusquement l'arc primaire. De même, le moyen de freinage est muni d'un deuxième frein, hydraulique ou électrique, pour immobiliser brusquement l'arc secondaire.

Selon un troisième mode de réalisation, le moyen de freinage comporte au moins une première butée mécanique pour immobiliser brusquement l'arc primaire.

Cette première butée mécanique, disposée sur un côté latéral de la colonne immobile, est munie d'un anneau et d'un amortisseur. A l'issue du mouvement de l'arc primaire, une dent d'un moyen d'accrochage de ce dernier coopère avec l'anneau de manière à bloquer l'arc primaire, l'amortisseur faisant alors office de point d'arrêt.

De même, le moyen de freinage comporte une deuxième butée mécanique pour immobiliser brusquement ledit arc secondaire.

Cette deuxième butée mécanique est munie d'au moins un crochet et d'au moins un amortisseur agencés sur la première extrémité de l'arc primaire. A l'issue du mouvement de l'arc secondaire, le crochet coopère avec un moyen d'arrêt disposé sur la troisième extrémité de l'arc secondaire pour bloquer l'arc secondaire, l'amortisseur faisant alors office de point d'arrêt.

La rotation de l'arc secondaire autour du deuxième axe de rotation peut alors être réalisée sur pratiquement 360°. Néanmoins, certaines manipulations consistant à faire tourner plusieurs fois le patient sur lui-même, le moyen d'arrêt est avantageusement escamotable.

Par ailleurs, pour le confort du patient, l'arc secondaire est muni d'au moins un repose pied qui est solidaire de l'assise. La position le long du deuxième axe de rotation de l'ensemble repose pied/assise est donc réglable à l'aide du premier vérin. De plus, l'inclinaison du repose pied par rapport au deuxième axe de rotation est aussi ajustable.

Pareillement, l'arc secondaire possède un dossier réglable en hauteur le long du deuxième axe de rotation qui est soit solidaire de l'ensemble repose pied/assise soit réglable individuellement par un deuxième vérin par exemple.

De même, le fauteuil de consultation médicale comporte de préférence, une têtière inclinable et réglable en translation selon les premier et deuxième axes de rotation à l'aide d'un troisième vérin, d'une molette et d'un doigt d'indexage par exemple.

En outre, le patient devant être mis en mouvement, le fauteuil est muni d'au moins un moyen de contention permettant de solidariser le patient au fauteuil au niveau de sa tête, de son tronc, de son bassin et de ses membres inférieurs notamment au niveau des pieds.

Ces moyens de contention comportent, par exemple, des accoudoirs, des épaulements, un arceau de tête, des sangles, des poignées et un harnais réglables.

Dans une variante informatisée, le fauteuil est muni d'au moins deux capteurs de position et d'au moins deux capteurs de mouvement, chaque type de capteurs étant agencé sur l'arc primaire et l'arc secondaire par exemple, reliés à un organe de commande et de contrôle du fauteuil, pourvu d'un écran de visualisation.

Les capteurs de position permettent de vérifier si le centrage est correctement réalisé. Quant à eux, les capteurs de mouvement mesurent la vitesse et l'accélération angulaire ainsi que la totalité du déplacement angulaire réalisé.

La manipulation peut alors être entièrement assistée par l'organe de commande qui, lorsque le fauteuil est motorisé, contrôle les premier et deuxième moteur en s'aidant notamment des informations transmises par les capteurs. De plus, si le fauteuil est pourvu des premier et deuxième freins, ces derniers peuvent aussi être contrôlés par l'organe de commande.

Un praticien peut donc utiliser la présente invention pour mobiliser un patient et arrêter brusquement le mouvement entrepris de manière à traiter les vertiges positionnels. Pour limiter l'encombrement du cabinet du praticien, le fauteuil selon l'invention doit aussi être en mesure de remplir le rôle des différents équipements existants.

Ainsi, il comporte un premier et un deuxième moyen d'immobilisation respectivement de l'arc primaire par rapport à la colonne immobile et de l'arc secondaire, dans une pluralité de position, par rapport à l'arc primaire.

De plus, le fauteuil comporte un système de vidéonystagmoscopie qui examine les yeux du patient sous lumière infrarouge afin de visualiser sur un écran les déplacements oculaires réflexes, dénommés nystagmus par l'homme du métier, lors des divers mouvements du corps. Ce système de caméras peut être éventuellement connecté avec ou sans fils, par transmission hautes fréquences, à l'organe de commande et de gestion qui est alors pourvu d'un dispositif d'analyse du nystagmus.

L'invention et ses avantages apparaîtront avec plus de détails dans le cadre de la description suivante, qui illustre un exemple de réalisation préféré, donné sans aucun caractère limitatif, en référence aux figures annexées qui représentent :
- la figure 1, une vue de côté du fauteuil de consultation selon l'invention,
- la figure 2, une vue de dessous de la deuxième butée mécanique, et
- les figures 3,4 et 5, des schémas explicitant les modes de fonctionnement du fauteuil de consultation.

Les éléments présents dans plusieurs figures distinctes sont affectés d'une seule et même référence.

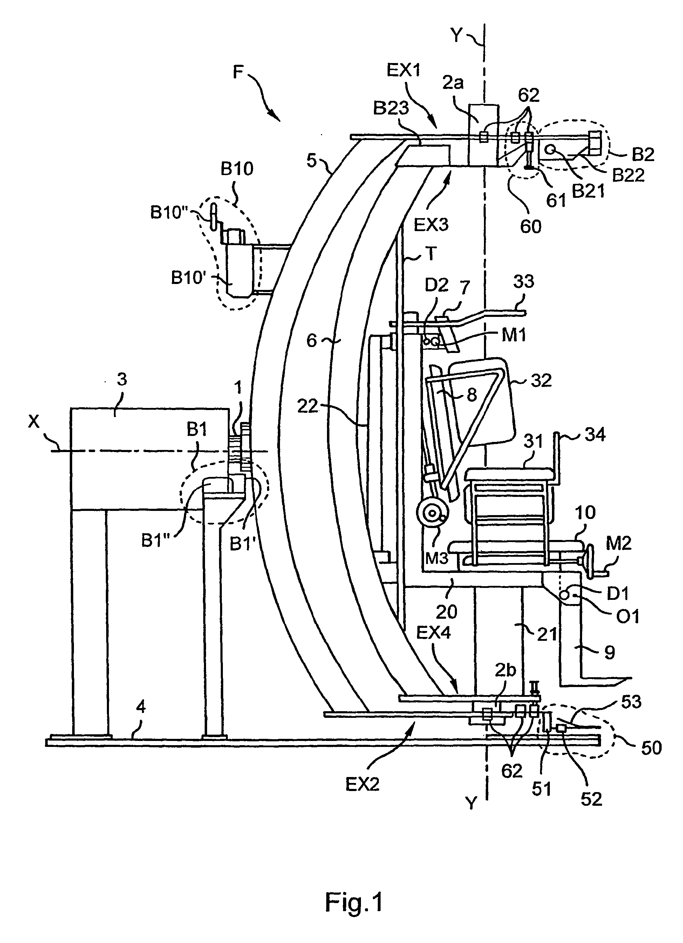

La figure 1 présente une vue de côté du fauteuil de consultation F selon l'invention.

L'ensemble du fauteuil F est fixé à une colonne immobile 3 par l'intermédiaire d'un arbre horizontal 1. Cette colonne immobile 3 est agencée sur un socle 4, reposant sur le sol, fortement lesté de manière à éviter toute bascule du fauteuil F, et ce quels que soient les mouvements réalisés et le poids du patient.

Bien évidemment, tout autre système de fixation de la colonne immobile, une fixation murale par exemple, est envisageable.

L'arc primaire 5, solidarisé à l'arbre horizontal 1 par le milieu de sa convexité, est capable d'effectuer un mouvement rotatif autour du premier axe de rotation X.

La position verticale de l'arc primaire 5, est éventuellement ajustable de manière à réaliser un centrage optimisé. Par suite, la colonne immobile 3 peut-être télescopique. De même, l'arc primaire 5 et/ou la colonne immobile 3 peuvent comporter un système de glissière à crémaillère ou tout autre système de réglage équivalent.

L'arc secondaire 6, légèrement plus petit que l'arc primaire 5, vient se loger au sein de ce dernier en se fixant sur les arbres supérieur 2a et inférieur 2b, disposés selon le deuxième axe de rotation Y passant par les première EX1 et deuxième EX2 extrémités de l'arc primaire. Ainsi, les arbres supérieur 2a et inférieur 2b permettent respectivement de relier la première extrémité EX1 à la troisième extrémité EX3 de l'arc secondaire et la deuxième extrémité EX2 à la quatrième extrémité EX4 de l'arc secondaire.

Par suite, l'arc secondaire 6 est à même d'effectuer un mouvement rotatif autour du deuxième axe de rotation Y.

Le fauteuil F comporte une ossature 20, agencée sur deux tiges T solidaires de l'arc secondaire 6, qui porte au moins un repose pied 9 et une assise 10. Un premier vérin 21, reposant sur la quatrième extrémité EX4, permet de régler la position de l'assise 10 et du repose pied 9 le long du deuxième axe de rotation Y.

De même, l'arc secondaire 6 comporte un dossier 8. La position de ce dernier peut éventuellement être réglable en translation selon le deuxième axe de rotation Y en coulissant le long des tiges T, via le premier vérin lorsque le dossier 8 est solidaire de l'ossature 20 ou à l'aide d'un deuxième vérin électrique lorsque dossier est indépendant de l'ossature 20.

De plus, l'inclinaison du repose pied 9 par rapport au deuxième axe de rotation Y est ajustable. Par suite, le repose pied possède une pluralité d'orifice O1, un doigt D1 venant s'emmancher dans un des orifices O1 pour bloquer le repose pied 9 dans une position précise.

Par ailleurs, le fauteuil F est muni d'une têtière 7 dont l'inclinaison et la position selon les premier X et deuxième Y axes de rotation est réglable. Pour ce faire, un troisième vérin 22, solidaire de l'ossature 20, participe au réglage de la position de la têtière 7 selon le deuxième axe de rotation Y. De même, un doigt d'indexage D2 règle la position de la têtière 7 selon le premier axe de rotation X et une molette M1 l'inclinaison de ce dernier, via des moyens usuels.

Par conséquent, le repose pied 9, l'assise 10, le dossier 8 et la têtière 7 disposent tous d'un degré de réglage suffisant pour s'adapter à la taille et à l'anatomie des patients.

En outre, le patient devant être mobilisé, il est solidarisé au fauteuil F par des moyens de contention à savoir des accoudoirs 31, des épaulements 32, un arceau de tête rembourrée 33, des poignées escamotables 34, un harnais et des sangles maintenant les membres inférieurs non représentés sur la figure 1.

Les accoudoirs 31 et les épaulements 32 sont adaptables à l'anatomie du patient en étant libres de se déplacer latéralement respectivement sous l'action de moyens usuels déclenchés par une première M2 et une deuxième M3 manivelles.

De même, la position de l'arceau de tête 33 selon le premier axe de rotation X est ajustable via un moyen usuel.

Par ailleurs, pour que le fauteuil F soit utilisé comme un fauteuil classique, il est impératif que tout mouvement rotatif de l'arc primaire 5 et/ou de l'arc secondaire 6 puisse être interdit.

Ainsi, le fauteuil est pourvu d'un premier 50 et d'un deuxième 60 moyen d'immobilisation respectivement des arcs primaire 5 et secondaire 6.

Le premier moyen d'immobilisation 50 est par exemple pourvu d'une plaque 51 munie d'un orifice, agencée sous la deuxième extrémité EX2 de l'arc primaire 5. Un doigt 52, mis en mouvement par une poignée 53, immobilise alors l'arc primaire 5 en étant inséré dans l'orifice de la plaque 51.

De son côté, le deuxième moyen d'immobilisation 60 est par exemple muni d'un doigt 61, solidaire de la troisième extrémité EX3 de l'arc secondaire 6, qui est inséré manuellement dans un orifice 62 de la première extrémité EX1 de l'arc primaire. Avantageusement, la première extrémité EX1 comporte une pluralité d'orifices 62 pour pouvoir autoriser l'immobilisation de l'arc secondaire 6 selon une pluralité de positions. De plus, ce deuxième moyen d'immobilisation 60 est doublé en étant aussi installé au niveau des deuxième EX2 et quatrième EX4 extrémités.

Par ailleurs, le praticien est à même de faire tourner les arcs primaire 5 et secondaire 6, respectivement autour des premier X et deuxième Y axes de rotation, manuellement, à l'aide de poignés par exemple, ou via un premier et un deuxième moteur non représentés. Afin d'obtenir une immobilisation brusque à l'issue d'un mouvement rotatif, le fauteuil F comporte un moyen de freinage.

Selon un premier mode de réalisation non représenté sur la figure 1, le moyen de freinage inclut les premier et deuxième moteurs qui en étant fortement démultipliés sont à même de s'arrêter instantanément.

Selon un deuxième mode de réalisation non représenté sur la figure 1, le moyen de freinage comporte des freins, hydraulique ou électrique, qui autorisent l'immobilisation brusque des arbres horizontal 1, supérieur 2a et/ou inférieur 2b, ce qui permet de stopper l'éventuel mouvement rotatif des arcs primaire 5 et/ou secondaire 6.

En référence à la figure 1, selon un troisième mode de réalisation, le moyen de freinage comporte une première B1 et une deuxième B2 butées mécaniques pour respectivement immobiliser les arcs primaire 5 et secondaire 6 à l'issue d'un mouvement rotatif.

La première butée mécanique B1 est agencée sur au moins un côté latéral de la colonne 3, et de préférence sur les deux côtés latéraux de cette dernière. Elle est munie d'un amortisseur B1' et d'un anneau B1". Un moyen d'accrochage B10, agencé sur l'arc primaire 5 et pourvu d'une plaque dentée B10" ainsi que d'une plaque d'arrêt B10', coopère avec la première butée B1 pour immobiliser brusquement l'arc primaire à l'issue d'un mouvement rotatif rapide de ce dernier. Par suite, la plaque d'arrêt B10' est brutalement bloquée par l'amortisseur B1', l'anneau B1" étant alors enserré par une dent de la plaque dentée B10". Un mouvement rapide de l'arc primaire 5 s'arrête donc brusquement ce qui génère une forte décélération.

De part l'agencement, représenté sur la figure 1, de ces premières butées mécaniques, l'arc primaire peut effectuer un mouvement rotatif autour du premier axe de rotation X sur amplitude de 180°, et plus précisément sur une amplitude allant de -90° à +90° par rapport la verticale. Néanmoins, en agençant différemment les butées mécaniques B1, d'autres amplitudes sont envisageables.

La deuxième butée mécanique B2 est agencée à la première extrémité EX1 de l'arc primaire 5. Elle comporte au moins un amortisseur B21 et au moins un crochet B22. A l'issue d'un mouvement rotatif rapide de l'arc secondaire 6 autour du deuxième axe de rotation Y, un moyen d'arrêt, une plaque B23 agencée sur la troisième extrémité EX3, coopère avec la deuxième butée B2. Ainsi, la plaque B23 est stoppée par l'amortisseur B22 et reste bloquée dans cette position en étant enserrée par le crochet B22.

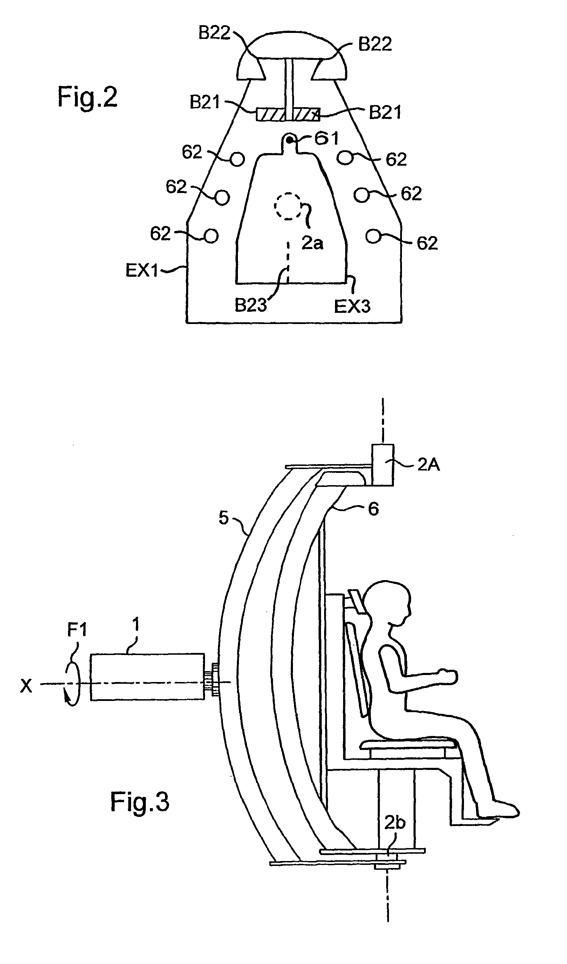

La figure 2 présente une vue de dessous des première et troisième extrémités montrant notamment la deuxième butée mécanique. L'arc secondaire 6 pouvant tourner dans le sens senestrorsum et dextrorsum, la deuxième butée mécanique comporte deux amortisseurs B21 et deux crochets B22.

La figure 2 fait aussi apparaître le doigt 61 et les orifices 62 permettant d'immobiliser l'arc secondaire 6.

Les figures 3,4 et 5 montrent de quelle manière un patient peut être mobilisé selon trois plans perpendiculaires.

En référence à la figure 3, l'arc secondaire 6 est immobilisé de manière à ce que le patient P tourne le dos à l'arbre horizontal 1. En faisant tourner l'arc primaire autour de l'axe X, on mobilise latéralement le patient en bloc d'un côté vers l'autre. Conformément à la flèche F1, le patient est mobilisé de sa gauche vers sa droite.

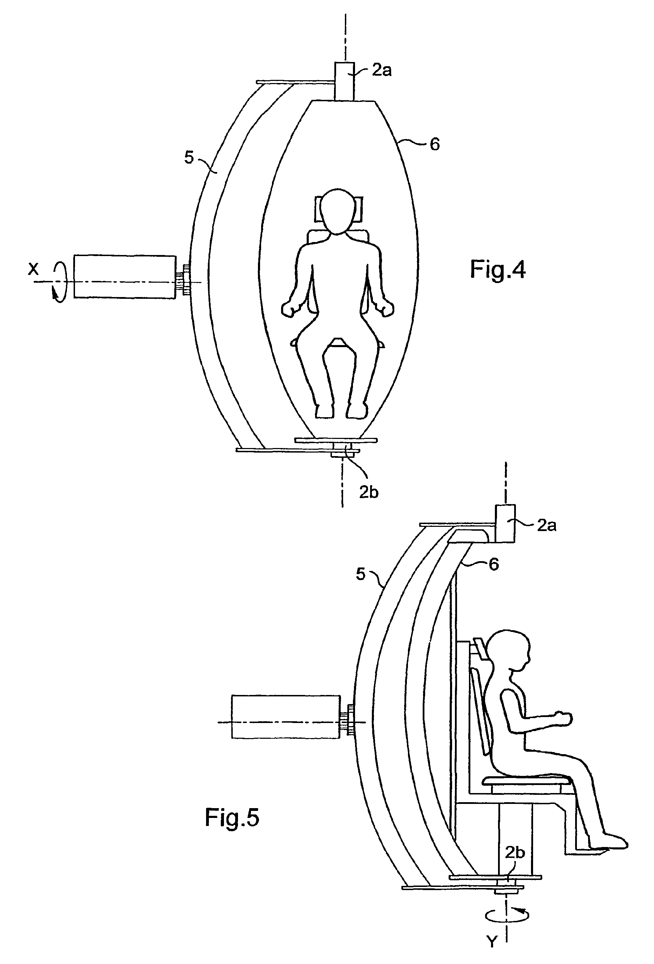

En référence à la figure 4, l'arc secondaire 6 est tourné de 90° puis immobilisé dans cette position. En faisant tourner l'arc primaire autour de l'axe X, on mobilise le patient en bloc d'avant en arrière.

En référence à la figure 5, l'arc primaire 5 est immobilisé en position verticale. En faisant tourner l'arc secondaire autour de l'axe Y, on mobilise le patient en bloc suivant l'axe de son tronc et de sa tête.

En réalisant les mouvement décrits précédemment et en les arrêtant brusquement à l'aide d'un moyen de freinage instantané, un praticien peut traiter les vertiges positionnels.

Le fauteuil de consultation F est donc particulièrement bien adapté à la réalisation, chez des patients présentant certains types de vertiges, de manipulations diagnostiques et thérapeutiques spécifiques, permettant une mobilisation en bloc selon trois plans perpendiculaires, soulageant ainsi l'effort physique réalisé par le praticien et augmentant le confort du patient manipulé. Il sera principalement utilisé par le médecin oto-rhino-laryngologiste ou le kinésithérapeute, mais aussi par tous les praticiens habilités et désirant prendre en charge ce type de pathologie vertigineuse.

Naturellement, la présente invention est sujette à de nombreuses variations quant à sa mise en oeuvre. On comprend bien qu'il n'est pas concevable d'identifier de manière exhaustive tous les modes de réalisations possibles. Il est bien sûr envisageable de remplacer un moyen décrit par un moyen équivalent sans sortir du cadre de la présente invention qui est définie seulement par les revendications.

## Revendications

1. Fauteuil de consultation médicale (F), pour asseoir et mobiliser un patient selon trois plans sensiblement perpendiculaires et sur une grande amplitude, comportant un arc primaire (5) relié à une colonne immobile (3) via un arbre horizontal (1) qui constitue un premier axe de rotation (X), un deuxième axe de rotation (Y), sensiblement perpendiculaire au premier axe de rotation (X), passant par une première (EX1) et une deuxième (EX2) extrémités dudit arc primaire (5),
**caractérisé en ce qu'**il comporte un arc secondaire (6), muni d'une assise (10), qui est agencé à l'intérieur dudit arc primaire (5) en étant respectivement fixé par une troisième (EX3) et une quatrième (EX4) extrémités aux dites premières (EX1) et deuxième (EX2) extrémités via un arbre supérieur (2a) et un arbre inférieur (2b), lesdits arcs primaire (5) et secondaire (6) étant aptes à respectivement effectuer un mouvement rotatif autour desdits premier (X) et deuxième (Y) axes de rotation, ledit fauteuil de consultation médicale (F) comportant en outre un moyen de freinage pour arrêter brusquement ledit mouvement rotatif.

2. Fauteuil selon la revendication 1,
**caractérisé en ce qu'**il comporte un premier moteur pour mettre en rotation ledit arc primaire (5) autour dudit premier axe de rotation (X).

3. Fauteuil selon l'une quelconque des revendications précédentes,
**caractérisé en ce qu'**il comporte un deuxième moteur pour mettre en rotation ledit arc secondaire (6) autour dudit deuxième axe de rotation (Y).

4. Fauteuil selon la revendication 2,
**caractérisé en ce que** ledit moyen de freinage inclut ledit premier moteur qui permet une immobilisation brusque dudit arc primaire (5).

5. Fauteuil selon la revendication 3,
**caractérisé en ce que** ledit moyen de freinage inclut ledit deuxième moteur qui permet une immobilisation brusque dudit arc secondaire (6).

6. Fauteuil selon l'une quelconque des revendications 1 à 3,
**caractérisé en ce que** ledit moyen de freinage comporte un premier frein pour immobiliser l'arc primaire (5).

7. Fauteuil selon l'une quelconque des revendications 1 à 3,
**caractérisé en ce que** ledit moyen de freinage comporte un deuxième frein pour immobiliser l'arc secondaire (6).

8. Fauteuil selon l'une quelconque des revendications 1 à 3,
**caractérisé en ce que** ledit moyen de freinage comporte au moins une première butée mécanique (B1) pour immobiliser brusquement ledit arc primaire (5) par rapport à ladite colonne immobile (3).

9. Fauteuil selon la revendication 8,
**caractérisé en ce que** ladite première butée mécanique (B1), disposée sur un côté latéral de ladite colonne immobile (3), est munie d'un anneau (B1") et d'un amortisseur (B1'), ledit anneau (B1") coopérant avec une dent d'un moyen d'accrochage (B10) agencé sur l'arc primaire (5).

10. Fauteuil selon l'une quelconque des revendications 1 à 3,
**caractérisé en ce que** ledit moyen de freinage comporte une deuxième butée mécanique (B2) pour immobiliser brusquement ledit arc secondaire (6) par rapport au dit arc primaire (5).

11. Fauteuil selon la revendication 10,
**caractérisé en ce que** ladite deuxième butée mécanique (B2) est munie d'au moins un crochet (B22) et d'au moins un amortisseur (B21) agencés sur la première extrémité (EX1) de l'arc primaire (5), ledit crochet (B22) coopérant avec un moyen d'arrêt (B23) disposé sur la troisième extrémité (EX3) de l'arc secondaire (6).

12. Fauteuil selon la revendication 11,
**caractérisé en ce que** ledit moyen d'arrêt (B23) est escamotable.

13. Fauteuil selon l'une quelconque des revendications précédentes,
**caractérisé en ce que** la position de ladite assise (10) le long dudit deuxième axe de rotation (Y) est réglable.

14. Fauteuil selon l'une quelconque des revendications précédentes,
**caractérisé en ce qu'**il comporte un dossier (8) réglable en translation selon ledit deuxième axe de rotation (Y).

15. Fauteuil selon l'une quelconque des revendications précédentes,
**caractérisé en ce qu'**il comporte au moins un repose pied (9) réglable en translation selon ledit deuxième axe de rotation (Y).

16. Fauteuil selon la revendication 15,
**caractérisé en ce que** l'inclinaison dudit repose pied (9) par rapport au deuxième axe de rotation (Y) est réglable.

17. Fauteuil selon l'une quelconque des revendications précédentes,
**caractérisé en ce qu'**il comporte une têtière (7) inclinable et réglable en translation selon lesdits premier (X) et deuxième (Y) axes de rotation.

18. Fauteuil selon l'une quelconque des revendications précédentes,
**caractérisé en ce qu'**il comporte au moins un moyen de contention (31,32,33,34) du patient.

19. Fauteuil selon l'une quelconque des revendications précédentes,
**caractérisé en ce qu'**il comporte un premier moyen d'immobilisation (50) de l'arc primaire (5) par rapport à la colonne immobile (3).

20. Fauteuil selon l'une quelconque des revendications précédentes,
**caractérisé en ce qu'**il comporte un deuxième moyen d'immobilisation (60), dans une pluralité de position, de l'arc secondaire (6) par rapport à l'arc primaire (5).

21. Fauteuil selon l'une quelconque des revendications précédentes,
**caractérisé en ce qu'**il comporte au moins deux capteurs de position.

22. Fauteuil selon l'une quelconque des revendications précédentes,
**caractérisé en ce qu'**il comporte au moins deux capteurs de mouvement.

23. Fauteuil selon l'une quelconque des revendications précédentes,
**caractérisé en ce qu'**il comporte un système de vidéonystagmoscopie.

24. Fauteuil selon l'une quelconque des revendications précédentes,
**caractérisé en ce qu'**il comporte un organe de commande et de gestion.

25. Fauteuil selon l'une quelconque des revendications précédentes,
**caractérisé en ce que** ledit arc primaire (5) est relié audit arbre horizontal (1) par le milieu de sa convexité.

## Claims

1. Medical examination chair (F) for seating and moving a patient in three substantially perpendicular planes and over a large range, comprising a primary arc (5), which is connected to a stationary column (3) via a horizontal shaft (1), which constitutes a first axis of rotation (X), a second axis of rotation (Y), substantially perpendicular to the first axis of rotation (X), passing through a first end (EX1) and a second end (EX2) of said primary arc (5), **characterised in that** it comprises a secondary arc (6), which is provided with a seat (10) and is arranged inside said primary arc (5), being secured via a third end (EX3) and a fourth end (EX4) to the aforementioned first end (EX1) and second end (EX2) respectively via an upper shaft (2a) and a lower shaft (2b), said primary arc (5) and secondary arc (6) being able respectively to perform a rotary movement about said first axis (X) and second axis (Y) of rotation, said medical examination chair (F) further comprising braking means for suddenly stopping said rotary movement.

2. Chair according to claim 1, **characterised in that** it comprises a first motor for setting said primary arc (5) into rotation about said first axis of rotation (X).

3. Chair according to either of the preceding claims, **characterised in that** it comprises a second motor for setting said secondary arc (6) into rotation about said second axis of rotation (Y).

4. Chair according to claim 2, **characterised in that** said braking means includes said first motor, which allows said primary arc (5) to be stopped suddenly.

5. Chair according to claim 3, **characterised in that** said braking means includes said second motor, which allows said secondary arc (6) to be stopped suddenly.

6. Chair according to any one of claims 1 to 3, **characterised in that** said braking means comprises a first brake for stopping the primary arc (5).

7. Chair according to any one of claims 1 to 3, **characterised in that** said braking means comprises a second brake for stopping the secondary arc (6).

8. Chair according to any one of claims 1 to 3, **characterised in that** said braking means comprises at least a first mechanical abutment (B1) for suddenly stopping said primary arc (5) relative to said stationary column (3).

9. Chair according to claim 8, **characterised in that** said first mechanical abutment (B1), which is placed on a lateral side of said stationary column (3), is provided with a ring (B1") and a damper (B1'), said ring (B1") cooperating with a tooth of a catch means (B10) arranged on the primary arc (5).

10. Chair according to any one of claims 1 to 3, **characterised in that** said braking means comprises a second mechanical abutment (B2) for suddenly stopping said secondary arc (6) relative to said primary arc (5).

11. Chair according to claim 10, **characterised in that** said second mechanical abutment (B2) is provided with at least one hook (B22) and at least one damper (B21) arranged on the first end (EX1) of the primary arc (5), said hook (B22) cooperating with a stop means (B23) which is disposed on the third end (EX3) of the secondary arc (6).

12. Chair according to claim 11, **characterised in that** said stop means (B23) is retractable.

13. Chair according to any one of the preceding claims, **characterised in that** the position of said seat (10) along said second axis of rotation (Y) is adjustable.

14. Chair according to any one of the preceding claims, **characterised in that** it comprises a seat back (8) which is adjustable in translation along said second axis of rotation (Y).

15. Chair according to any one of the preceding claims, **characterised in that** it comprises at least one foot rest (9) which is adjustable in translation along said second axis of rotation (Y).

16. Chair according to claim 15, **characterised in that** the inclination of said foot rest (9) relative to the second axis of rotation (Y) is adjustable.

17. Chair according to any one of the preceding claims, **characterised in that** it comprises a headrest (7) which is tiltable and adjustable in translation along said first axis (X) and second axis (Y) of rotation.

18. Chair according to any one of the preceding claims, **characterised in that** it comprises patient restraining means (31, 32, 33, 34).

19. Chair according to any one of the preceding claims, **characterised in that** it comprises a first locking means (50) for locking the primary arc (5) relative to the stationary column (3).

20. Chair according to any one of the preceding claims, **characterised in that** it comprises a second locking means (60) for locking the secondary arc (6) in a plurality of positions relative to the primary arc (5).

21. Chair according to any one of the preceding claims, **characterised in that** it comprises at least two position sensors.

22. Chair according to any one of the preceding claims, **characterised in that** it comprises at least two movement sensors.

23. Chair according to any one of the preceding claims, **characterised in that** it comprises a videonystagmoscopy system.

24. Chair according to any one of the preceding claims, **characterised in that** it comprises a command and control member.

25. Chair according to any one of the preceding claims, **characterised in that** said primary arc (5) is connected to said horizontal shaft (1) via the middle of the convex portion thereof.

## Patentansprüche

1. Medizinischer Behandlungsstuhl (F), um einen Patienten zu setzen und gemäß drei im Wesentlichen lotrechten Ebenen und über eine große Amplitude in Bewegung zu versetzen, der einen primären Bogen (5) aufweist, der über eine waagrechte Welle (1), die eine erste Drehachse (X) bildet, mit einer unbeweglichen Säule (3) verbunden ist, wobei eine zweite Drehachse (Y) im Wesentlichen lotrecht zur ersten Drehachse (X) über ein erstes (EX1) und ein zweites Ende (EX2) des primären Bogens (5) verläuft, **dadurch gekennzeichnet, dass** er einen mit einer Sitzfläche (10) versehenen sekundären Bogen (6) aufweist, der innerhalb des primären Bogens (5) angeordnet ist, indem er über ein drittes (EX3) bzw. viertes Ende (EX4) am ersten (EX1) bzw. zweiten Ende (EX2) über eine obere Welle (2a) bzw. eine untere Welle (2b) befestigt ist, wobei der primäre Bogen (5) und der sekundäre Bogen (6) fähig sind, je eine Drehbewegung um die erste (X) bzw. zweite Drehachse (Y) auszuführen, wobei der medizinische Behandlungsstuhl (F) außerdem ein Bremsmittel aufweist, um die Drehbewegung plötzlich anzuhalten.

2. Stuhl nach Anspruch 1, **dadurch gekennzeichnet, dass** er einen ersten Motor aufweist, um den primären Bogen (5) um die erste Drehachse (X) in Drehung zu versetzen.

3. Stuhl nach einem der vorhergehenden Ansprüche, **dadurch gekennzeichnet, dass** er einen zweiten Motor aufweist, um den sekundären Bogen (6) um die zweite Drehachse (Y) in Drehung zu versetzen.

4. Stuhl nach Anspruch 2, **dadurch gekennzeichnet, dass** das Bremsmittel den ersten Motor umfasst, der einen plötzlichen Stillstand des primären Bogens (5) erlaubt.

5. Stuhl nach Anspruch 3, **dadurch gekennzeichnet, dass** das Bremsmittel den zweiten Motor umfasst, der einen plötzlichen Stillstand des sekundären Bogens (6) erlaubt.

6. Stuhl nach einem der Ansprüche 1 bis 3, **dadurch gekennzeichnet, dass** das Bremsmittel eine erste Bremse aufweist, um den primären Bogen (5) anzuhalten.

7. Stuhl nach einem der Ansprüche 1 bis 3, **dadurch gekennzeichnet, dass** das Bremsmittel eine zweite Bremse aufweist, um den sekundären Bogen (6) anzuhalten.

8. Stuhl nach einem der Ansprüche 1 bis 3, **dadurch gekennzeichnet, dass** das Bremsmittel mindestens einen ersten mechanischen Anschlag (B 1) aufweist, um den primären Bogen (5) bezüglich der unbeweglichen Säule (3) plötzlich anzuhalten.

9. Stuhl nach Anspruch 8, **dadurch gekennzeichnet, dass** der erste mechanische Anschlag (B1), der auf einer Seitenfläche der unbeweglichen Säule (3) angeordnet ist, mit einem Ring (B1") und einem Dämpfer (B1') versehen ist, wobei der Ring (B1") mit einem Zahn eines Befestigungsmittels (B 10) zusammenwirkt, das auf dem primären Bogen (5) angeordnet ist.

10. Stuhl nach einem der Ansprüche 1 bis 3, **dadurch gekennzeichnet, dass** das Bremsmittel einen zweiten mechanischen Anschlag (B2) aufweist, um den sekundären Bogen (6) bezüglich des primären Bogens (5) plötzlich anzuhalten.

11. Stuhl nach Anspruch 10, **dadurch gekennzeichnet, dass** der zweite mechanische Anschlag (B2) mit mindestens einem Haken (B22) und mit mindestens einem Dämpfer (B21) versehen ist, die auf dem ersten Ende (EX1) des primären Bogens (B23) angeordnet sind, wobei der Haken (B22) mit einem Haltemittel (B23) zusammenwirkt, das auf dem dritten Ende (EX3) des sekundären Bogens (6) angeordnet ist.

12. Stuhl nach Anspruch 11, **dadurch gekennzeichnet, dass** das Haltemittel (B23) einziehbar ist.

13. Stuhl nach einem der vorhergehenden Ansprüche, **dadurch gekennzeichnet, dass** die Stellung der Sitzfläche (10) entlang der zweiten Drehachse (Y) einstellbar ist.

14. Stuhl nach einem der vorhergehenden Ansprüche, **dadurch gekennzeichnet, dass** er eine Rückenlehne (8) aufweist, die in Translationsrichtung gemäß der zweiten Drehachse (Y) einstellbar ist.

15. Stuhl nach einem der vorhergehenden Ansprüche, **dadurch gekennzeichnet, dass** er mindestens eine Fußstütze (9) aufweist, die in Translationsrichtung gemäß der zweiten Drehachse (Y) einstellbar ist.

16. Stuhl nach Anspruch 15, **dadurch gekennzeichnet, dass** die Neigung der Fußstütze (9) bezüglich der zweiten Drehachse (Y) einstellbar ist.

17. Stuhl nach einem der vorhergehenden Ansprüche, **dadurch gekennzeichnet, dass** er ein Kopfstück (7) aufweist, das neigbar und in Translationsrichtung gemäß der ersten (X) und der zweiten Drehachse (Y) einstellbar ist.

18. Stuhl nach einem der vorhergehenden Ansprüche, **dadurch gekennzeichnet, dass** er mindestens ein Mittel (31, 32, 33, 34) zum Fixieren des Patienten aufweist.

19. Stuhl nach einem der vorhergehenden Ansprüche, **dadurch gekennzeichnet, dass** er ein erstes Mittel (50) zum Anhalten des primären Bogens (5) bezüglich der unbeweglichen Säule (3) aufweist.

20. Stuhl nach einem der vorhergehenden Ansprüche, **dadurch gekennzeichnet, dass** er ein zweites Mittel (60) zum Anhalten des sekundären Bogens (6) bezüglich des primären Bogens (5) in mehreren Stellungen aufweist.

21. Stuhl nach einem der vorhergehenden Ansprüche, **dadurch gekennzeichnet, dass** er mindestens zwei Positionssensoren aufweist.

22. Stuhl nach einem der vorhergehenden Ansprüche, **dadurch gekennzeichnet, dass** er mindestens zwei Bewegungssensoren aufweist.

23. Stuhl nach einem der vorhergehenden Ansprüche, **dadurch gekennzeichnet, dass** er ein Video-Nystagmoskopie-System aufweist.

24. Stuhl nach einem der vorhergehenden Ansprüche, **dadurch gekennzeichnet, dass** er ein Steuer- und Verwaltungsorgan aufweist.

25. Stuhl nach einem der vorhergehenden Ansprüche, **dadurch gekennzeichnet, dass** der primäre Bogen (5) mit der waagrechten Welle (1) über die Mitte seiner Wölbung verbunden ist.
